(19) Europäisches Patentamt
European Patent Office
Office européen des brevets

(11) Veröffentlichungsnummer: **0 490 220 A1**

(12) **EUROPÄISCHE PATENTANMELDUNG**

(21) Anmeldenummer: **91120708.2**

(51) Int. Cl.5: **C07D 317/46**, A01N 43/30

(22) Anmeldetag: **02.12.91**

(30) Priorität: **14.12.90 DE 4040021**

(43) Veröffentlichungstag der Anmeldung:
**17.06.92 Patentblatt 92/25**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Knüppel, Peter C., Dr.**
**Staelsmühle 13**
**W-5632 Wermelskirchen 3(DE)**
Erfinder: **Marhold, Albrecht, Dr.**
**Carl-Duisberg-Strasse 329**
**W-5090 Leverkusen 1(DE)**
Erfinder: **Hausner, Thomas Peter, Dr.**
**Pützlachstrasse 91**
**W-5000 Köln 80(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5090 Bergisch Gladbach 2(DE)**
Erfinder: **Schmidt, Robert R., Dr.**
**Im Waldwinkel 110**
**W-5060 Bergisch Gladbach 2(DE)**
Erfinder: **Dehne, Heinz-Wilhelm, Dr.**
**Krischer Strasse 81**
**W-4019 Monheim(DE)**

(54) **Substituierte 2,2-Difluor-1,3-benzodioxyl-4-ketone.**

(57) Die Erfindung betrifft neue substituierte 2,2-Difluor-1,3-benzodioxyl-4-ketone der allgemeinen Formel (I)

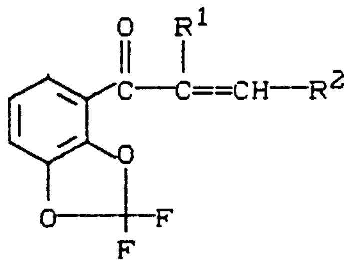

(I)

in welcher

$R^1$ für eine Gruppe aus der Reihe -CN; $-CO_2R^5$ oder

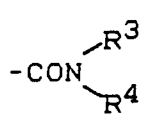

steht,
worin

$R^3$ und $R^4$ jeweils unabhängig voneinander für Wasserstoff, Alkyl, Alkenyl, Alkylcarbonyl, Formyl oder

EP 0 490 220 A1

gegebenenfalls substituiertes Aralkyl stehen,

$R^5$ für Alkyl steht und

$R^2$ für eine Gruppe aus der Reihe

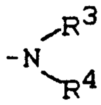

oder $-OR^3$ steht,

worin

$R^3$ und $R^4$ die oben angegebene Bedeutung haben,

ein Verfahren zu ihrer Herstellung und ihre Verwendung als Pflanzenschutzmittel, insbesondere als Herbizide.

Die vorliegende Erfindung betrifft neue 2,2-Difluor-1,3-benzodioxyl-4-ketone, ein Verfahren zu ihrer Herstellung und ihre Verwendung als Pflanzenschutzmittel, insbesondere als Herbizide.

Es ist bereits bekannt, daß bestimmte substituierte Acrylnitrilderivate herbizide Eigenschaften besitzen (vergleiche DE-OS 23 30 913).

Die herbizide Wirkung dieser Verbindungen ist jedoch insbesondere bei niedrigen Aufwandmengen und -konzentrationen nicht in allen Anwendungsbereichen völlig zufriedenstellend.

Es wurden neue substituierte 2,2-Difluor-1,3-benzodioxyl-4-ketone der allgemeinen Formel (I)

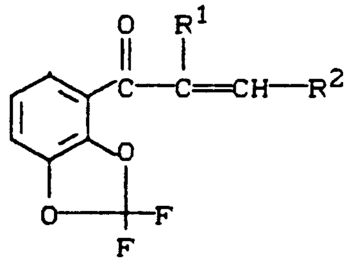

(I)

in welcher

$R^1$ für eine Gruppe aus der Reihe -CN; -$CO_2R^5$ oder

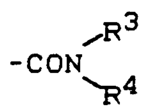

steht, worin

$R^3$ und $R^4$ jeweils unabhängig voneinander für Wasserstoff, Alkyl, Alkenyl, Alkylcarbonyl, Formyl oder gegebenenfalls substituiertes Aralkyl stehen,

$R^5$ für Alkyl steht und

$R^2$ für eine Gruppe aus der Reihe

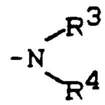

oder -$OR^3$ steht,

worin

$R^3$ und $R^4$ die oben angegebene Bedeutung haben,

gefunden.

Weiterhin wurde gefunden, daß man die neuen substituierten 2,2-Difluor-1,3-benzodioxyl-4-ketone der allgemeinen Formel (I)

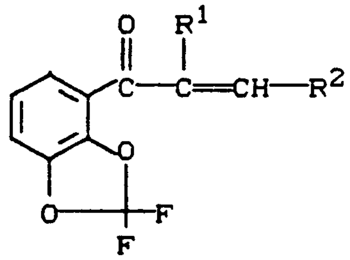

(I)

in welcher

$R^1$ und $R^2$ die oben angegebenen Bedeutungen haben, erhält,

wenn man das Säurechlorid der Formel (II)

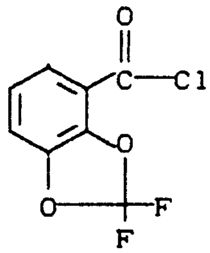

(II)

mit substituierten Acrylsäurederivaten der Formel (III)

$R^1$-CH = CH-$R^2$ (III)

in welcher
$R^1$ und $R^2$ die oben angegebenen Bedeutungen haben,
gegebenenfalls in Gegenwart eines Verdünnungsmittels und gegebenenfalls in Gegenwart einer Base umsetzt.

Schließlich wurde gefunden, daß die neuen substituierten 2,2-Difluor-1,3-benzodioxyl-4-ketone der Formel (I) eine sehr gute herbizide Wirksamkeit besitzen.

Vorzugsweise bedeutet in den allgemeinen Formeln im folgenden, falls nicht anders definiert:
Alkyl, einzeln oder in zusammengesetzten Resten

- geradkettiges oder verzweigtes Alkyl mit 1 bis 6, insbesondere 1 bis 4 Kohlenstoffatomen. Beispielhaft und vorzugsweise seien Methyl, Ethyl, n- oder i-Propyl, n-, i-, s- und t-Butyl, genannt.

Alkenyl

- geradkettiges oder verzweigtes Alkenyl mit vorzugsweise 2 bis 6, besonders bevorzugt 2 bis 4 und insbesondere 3 Kohlenstoffatomen. Beispielhaft seien Vinyl, Allyl, Propenyl-(2), Butenyl-(1), 2-Butenyl, 3-Butenyl und 1-Methallyl genannt.

Aryl

- unsubstituiertes oder substituiertes Aryl mit 6 bis 10 Kohlenstoffatomen. Beispielhaft und vorzugsweise sei jeweils unsubstituiertes oder substituiertes Phenyl und Naphthyl, insbesondere unsubstituiertes oder substituiertes Phenyl genannt.

Die erfindungsgemäßen neuen substituierten 2,2-Difluor-1,3-benzodioxyl-4-ketone sind durch die Formel (I) allgemein definiert. Bevorzugt sind Verbindungen der Formel (I), in welchen

$R^1$ für eine Gruppe aus der Reihe -CN; -$CO_2R^5$ oder

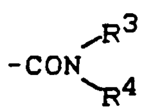

steht,
worin

$R^3$ und $R^4$ jeweils unabhängig voneinander für Wasserstoff, geradkettiges oder verzweigtes Alkyl mit 1 bis 4 Kohlenstoffatomen, geradkettiges oder verzweigtes Alkenyl mit 2 bis 4 Kohlenstoffatomen, geradkettiges oder verzweigtes Alkylcarbonyl mit 1 bis 4 Kohlenstoffatomen, Formyl oder gegebenenfalls für einfach oder zweifach durch Fluor, Chlor, Brom, Methyl, Methoxy, Trifluormethyl oder Trifluormethoxy substituiertes Benzyl stehen und

$R^5$ für geradkettiges oder verzweigtes Alkyl mit 1 bis 4 Kohlenstoffatomen steht und

$R^2$ für eine Gruppe aus der Reihe

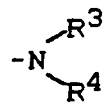

oder $-OR^3$ steht,

worin

$R^3$ und $R^4$ die oben angegebene Bedeutung haben.

Ganz besonders bevorzugt sind Verbindungen der Formel (I), in welchen

$R^1$ für eine Gruppe aus der Reihe -CN, $-CO_2R^5$ oder

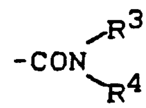

steht,

wobei

$R^3$ und $R^4$ jeweils unabhängig voneinander für Wasserstoff, Methyl, Ethyl, n- oder i-Propyl, Vinyl, Allyl, Methylcarbonyl, Ethylcarbonyl, Formyl oder Benzyl stehen und

$R^5$ für Methyl oder Ethyl steht und

$R^2$ für eine Gruppe aus der Reihe

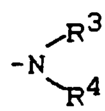

oder $-OR^3$ steht,

worin

$R^3$ und $R^4$ die oben angegebene Bedeutung haben.

Im einzelnen seien außer den bei den Herstellungsbeispielen genannten Verbindungen die folgenden substituierten 2,2-Difluor-1,3-benzodioxyl-4-ketone der allgemeinen Formel (I) genannt:

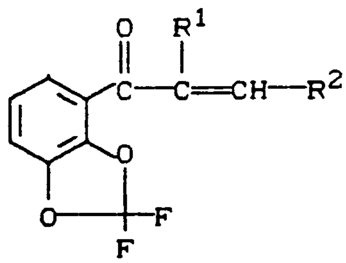

(I)

Tabelle 1:

| $R^1$ | $R^2$ |
|---|---|
| CN | $-NHCH_3$ |
| CN | 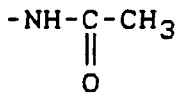 |
| CN | $-NH-C_2H_5$ |
| CN | 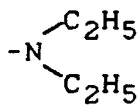 |
| CN | $-NH_2$ |
| CN | 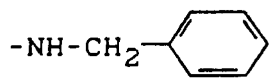 |
| $CO_2CH_3$ | 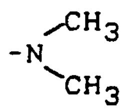 |
| $CO_2C_2H_5$ | 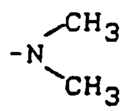 |
| $CO_2NH_2$ | 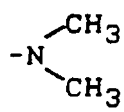 |
| $CO_2CH_3$ | 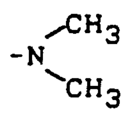 |

Verwendet man beispielsweise 2,2-Difluor-1,3-benzodioxol-4-carbonsäurechlorid und N,N-Dimethylaminoacrylsäurenitril als Ausgangsstoffe und Triethylamin als Base, so läßt sich das erfindungsgemäße Verfahren durch das folgende Formelschema darstellen:

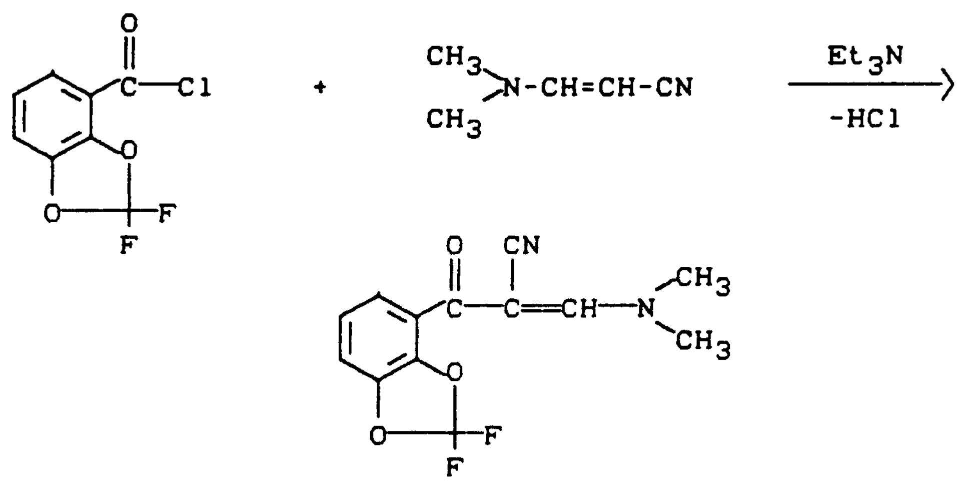


Das bei dem erfindungsgemäßen Verfahren als Ausgangsstoff zu verwendende Säurechlorid der Formel (II) ist neu, kann aber nach allgemein bekannten und üblichen Verfahren erhalten werden, wenn man die Carbonsäure der Formel (IV)

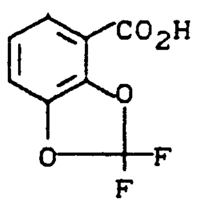

(IV)

mit Halogenierungsmitteln, wie beispielsweise Thionylchlorid oder Oxalsäuredichlorid gegebenenfalls in Gegenwart eines Verdünnungsmittels, wie beispielsweise Benzol, bei Temperaturen zwischen 50 ° C und 100 ° C gegebenenfalls in Gegenwart einer Stickstoff-Atmosphäre erhitzt (vergleiche beispielsweise "Reaktionen und Synthesen im organisch-chemischen Praktikum", L.-F. Tietze, T. Eicher, Georg Thieme Verlag Stuttgart, New York 1981).

Die Carbonsäure der Formel (IV) ist bekannt (vergleiche EP-OS 0 333 658).

Die bei der Durchführung des erfindungsgemäßen Verfahrens außerdem zu verwendenden substituierten Acrylsäurederivate der Formel (III) sind durch die Formel (III) allgemein definiert. In der Formel (III) haben $R^1$ und $R^2$ vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Verbindungen der Formel (III) sind allgemein bekannte Verbindungen der organischen Chemie.

Das erfindungsgemäße Verfahren zur Herstellung der neuen substituierten 2,2-Difluor-1,3-benzodioxyl-4-ketone der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calciumhydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 120 °C, vorzugsweise bei Temperaturen zwischen 60 °C und 100 °C.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei eignen sich die erfindungsgemäßen Wirkstoffe der Formel (I) besonders gut zur selektiven Bekämpfung von monokotylen und dikotylen Unkräutern in monokotylen und dikotylen Kulturen im Vor- und Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); Methyl6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amino-4-chlor-2-phenyl-2,3-dihydro-3-oxypyridazin (CHLORIDAZON); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [-(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octylthiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN); in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden, Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 2 kg pro ha.

Die Herstellung und die Verwendung der erfindungsgemäßen Wirkstoffe geht aus den nachfolgenden Beispielen hervor.

Herstellungsbeispiel:

Beispiel 1:

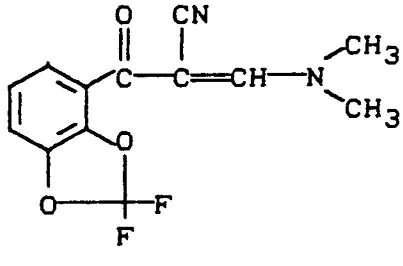

13,1 g (0,059 Mol) 2,2-Difluor-1,3-benzodioxol-4-carbonsäurechlorid werden in 75 ml Dioxan gelöst. Zu dieser Lösung werden bei Raumtemperatur 5,7 g (0,059 Mol) N,N-Dimethylaminoacrylsäurenitril zugetropft. Das Reaktionsgemisch wird eine Stunde bei Raumtemperatur und 16 Stunden bei Rückflußtemperatur gerührt. Nach dem Abkühlen wird der Feststoff abgesaugt und die Mutterlauge unter vermindertem Druck eingedampft. Der Rückstand wird in Dichlormethan aufgenommen, mit Wasser gewaschen und getrocknet. Der nach dem Abdestillieren des Lösungsmittels verbleibende Rückstand wird mit Diisopropylether verrührt und getrocknet.

Man erhält 12,3 g (74 % der Theorie) 2,2-Difluor-1,3-benzodioxyl-4-(1-cyano-2-dimethylamino-vinyl)-keton mit einem Schmelzpunkt von 89 ° C bis 90 ° C.

In analoger Weise und unter Berücksichtigung der Angaben in der Beschreibung erhält man die nachfolgend in Tabelle 2 aufgeführten Endprodukte der Formel (I).

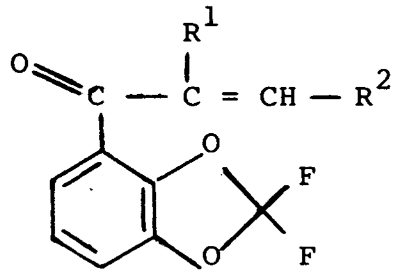

(I)

Tabelle 2

| Bsp. | $R^1$ | $R^2$ | $^1$H-NMR* |
|---|---|---|---|
| 2 | $-CO_2CH_3$ | $-N(CH_3)_2$ | 2,31; 2,86; 3,55 |
| 3 | $-CO_2C_2H_5$ | $-N(CH_3)_2$ | 0,94; 2,89; 3,32; 4,00 |

* Die $^1$H-NMR-Spektren wurden in Deuterochloroform ($CDCl_3$) oder Hexadeuterodimethylsulfoxid (DMSO- $d_6$) mit Trimethylsilan (TMS) als innerem Standard aufgenommen. Angegeben ist die chemische Verschiebung als $\delta$-Wert in ppm.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigt in diesem Test z.B. die Verbindung gemäß des Herstellungsbeispieles 1.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigt in diesem Test z.B. die Verbindung gemäß des Herstellungsbeispieles 1.

Beispiel C

Venturia-Test (Apfel) / protektiv

Lösungsmittel: 4,7 Gew.-Teile Aceton
Emulgator: 0,3 Gew.-Teile Alkyl-aryl-polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 ° C und 100 % relativer Luftfeuchtigkeit in einer Inkubationskabine.

Die Pflanzen werden dann im Gewächshaus bei 20 ° C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test die Verbindungen gemäß Herstellungsbeispiele.

**Patentansprüche**

**1.** Substituierte 2,2-Difluor-1,3-benzodioxyl-4-ketone der allgemeinen Formel (I)

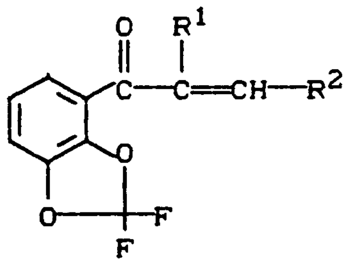

(I)

in welcher

$R^1$ für eine Gruppe aus der Reihe -CN; $-CO_2R^5$ oder

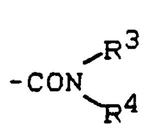

steht, worin

$R^3$ und $R^4$ jeweils unabhängig voneinander für Wasserstoff, Alkyl, Alkenyl, Alkylcarbonyl, Formyl oder gegebenenfalls substituiertes Aralkyl stehen,

$R^5$ für Alkyl steht und

$R^2$ für eine Gruppe aus der Reihe

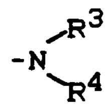

oder $-OR^3$ steht,
worin
$R^3$ und $R^4$ die oben angegebene Bedeutung haben.

**2.** Substituierte 2,2-Difluor-1,3-benzodioxyl-4-ketone der allgemeinen Formel (I) gemäß Anspruch 1, in welcher
$R^1$ für eine Gruppe aus der Reihe -CN; $-CO_2R^5$ oder

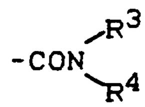

steht,
worin
$R^3$ und $R^4$ jeweils unabhängig voneinander für Wasserstoff, geradkettiges oder verzweigtes Alkyl mit 1 bis 4 Kohlenstoffatomen, geradkettiges oder verzweigtes Alkenyl mit 2 bis 4 Kohlenstoffatomen, geradkettiges oder verzweigtes Alkylcarbonyl mit 1 bis 4 Kohlenstoffatomen, Formyl oder gegebenenfalls für einfach oder zweifach durch Fluor, Chlor, Brom, Methyl, Methoxy, Trifluormethyl oder Trifluormethoxy substituiertes Benzyl stehen und
$R^5$ für geradkettiges oder verzweigtes Alkyl mit 1 bis 4 Kohlenstoffatomen steht und
$R^2$ für eine Gruppe aus der Reihe

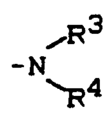

oder $-OR^3$ steht,
worin
$R^3$ und $R^4$ die oben angegebene Bedeutung haben.

**3.** Substituierte 2,2-Difluor-1,3-benzodioxyl-4-ketone der allgemeinen Formel (I) gemäß Anspruch 1, in welcher
$R^1$ für eine Gruppe aus der Reihe -CN, $-CO_2R^5$ oder

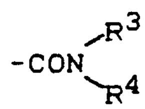

steht, wobei
$R^3$ und $R^4$ jeweils unabhängig voneinander für Wasserstoff, Methyl, Ethyl, n- oder i-Propyl, Vinyl, Allyl, Methylcarbonyl, Ethylcarbonyl, Formyl oder Benzyl stehen und
$R^5$ für Methyl oder Ethyl steht und
$R^2$ für eine Gruppe aus der Reihe

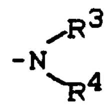

oder $-OR^3$ steht,
worin
$R^3$ und $R^4$ die oben angegebene Bedeutung haben.

**4.** Verfahren zur Herstellung substituierter 2,2-Difluor-1,3-benzodioxyl-4-ketone der allgemeinen Formel (I)

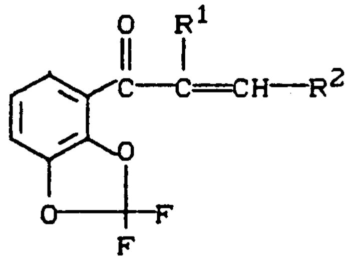

(I)

in welcher
$R^1$ für eine Gruppe aus der Reihe -CN; $-CO_2R^5$ oder

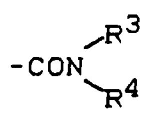

steht,
worin
$R^3$ und $R^4$ jeweils unabhängig voneinander für Wasserstoff, Alkyl, Alkenyl, Alkylcarbonyl, Formyl oder gegebenenfalls substituiertes Aralkyl stehen,
$R^5$ für Alkyl steht und
$R^2$ für eine Gruppe aus der Reihe

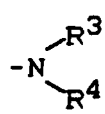

oder $-OR^3$ steht,
worin
$R^3$ und $R^4$ die oben angegebene Bedeutung haben,
dadurch gekennzeichnet, daß man das Säurechlorid der Formel (II)

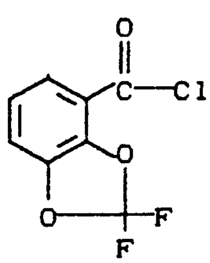

(II)

mit substituierten Acrylsäurederivaten der Formel (III)

$R^1$-CH = CH-$R^2$ (III)

in welcher
$R^1$ und $R^2$ die oben angegebenen Bedeutungen haben,
gegebenenfalls in Gegenwart eines Verdünnungsmittels und gegebenenfalls in Gegenwart einer Base umsetzt.

**5.** Herbizide Mittel, gekennzeichnet durch einen Gehalt an mindestens einem substituierten 2,2-Difluor-1,3-benzodioxyl-4-keton der allgemeinen Formel (I) gemäß den Ansprüchen 1 bis 4.

**6.** Verfahren zur Bekämpfung von Unkräutern, dadurch gekennzeichnet, daß man substituierte 2,2-Difluor-1,3-benzodioxyl-4-ketoneder allgemeinen Formel (I) gemäß den Ansprüchen 1 bis 4 auf Unkräuter und/oder ihren Lebensraum einwirken läßt.

**7.** Verwendung von substituierten 2,2-Difluor-1,3-benzodioxyl-4-ketonen der allgemeinen Formel (I) gemäß den Ansprüchen 1 bis 4 zur Bekämpfung von Unkräutern.

**8.** Verfahren zur Herstellung von herbiziden Mitteln, dadurch gekennzeichnet, daß man substituierte 2,2-Difluor-1,3-benzodioxyl-4-ketone der allgemeinen Formel (I) gemäß den Ansprüchen 1 bis 4 mit Streckmitteln und/oder oberflächenaktiven Substanzen vermischt.

**9.** (2,3-Difluormethylendioxy)benzoesäurechlorid der Formel (II)

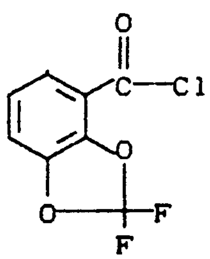

(II)

Europäisches Patentamt

# EUROPÄISCHER RECHERCHENBERICHT

Nummer der Anmeldung

EP 91 12 0708

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A<br>X | EP-A-0 333 658 (CIBA-GEIGY)<br>* Seite 2 * | 1<br>9 | C07D317/46<br>A01N43/30 |
| D,A | DE-A-2 330 913 (HOFFMANN-LA ROCHE)<br>* Seite 1 - Seite 2 * | 1,5-8 | |
| A | EP-A-0 005 756 (BAYER)<br>* Seiten 1 - 2; Seite 4, letzter Absatz * | 1,5-8 | |

RECHERCHIERTE SACHGEBIETE (Int. Cl.5)

C07D

Der vorliegende Recherchenbericht wurde für alle Patentansprüche erstellt

| Recherchenort | Abschlußdatum der Recherche | Prüfer |
|---|---|---|
| DEN HAAG | 19 FEBRUAR 1992 | RUSSELL F. ENGLISH |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)